(19) Europäisches Patentamt
European Patent Office
Office européen des brevets

(11) Numéro de publication: **0 408 105 B1**

# (12) FASCICULE DE BREVET EUROPEEN

(45) Date de publication de fascicule du brevet: **11.01.95**

(51) Int. Cl.[6]: **C07D 333/12**, C08G 61/12, H01M 4/62, H01B 1/12

(21) Numéro de dépôt: **90201736.7**

(22) Date de dépôt: **29.06.90**

## (54) Thiophènes fluorés polymères conducteurs dérivés de ces thiophènes, procédé pour leur obtention et dispositifs contenant ces polymères.

(30) Priorité: **10.07.89 FR 8909368**

(43) Date de publication de la demande:
**16.01.91 Bulletin 91/03**

(45) Mention de la délivrance du brevet:
**11.01.95 Bulletin 95/02**

(84) Etats contractants désignés:
**AT BE CH DE DK ES FR GB GR IT LI LU NL SE**

(56) Documents cités:
**FR-A- 2 624 126**
**US-A- 3 052 691**
**US-A- 3 197 480**

(73) Titulaire: **SOLVAY (Société Anonyme)**
**Rue du Prince Albert, 33**
**B-1050 Bruxelles (BE)**

(72) Inventeur: **Buechner, Werner**
**Rue Mireille 23**
**F-91600 Savigny S/0 (FR)**
Inventeur: **Lemaire, Marc**
**Rue Château Gaillard, 23**
**F-69100 Villeurbanne (FR)**
Inventeur: **Roncali, Jean**
**Rue des Bruyères 10**
**F-93260 Les Lilas (FR)**
Inventeur: **Garreau, Robert**
**Rue Montfleury 12**
**F-95200 Sarcelles (FR)**
Inventeur: **Garnier, Françis**
**Villa Remy 17**
**F-94500 Champigny (FR)**
Inventeur: **Hannecart, Etienne**
**Arboretumlaan 38**
**B-1980 Tervuren (BE)**

(74) Mandataire: **Nichels, William et al**
**Solvay**
**Département de la Propriété Industrielle**
**Rue de Ransbeek, 310**
**B-1120 Bruxelles (BE)**

EP 0 408 105 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des thiophènes substitués par un radical aliphatique ou aromatique au moins partiellement fluoré. L'invention concerne également les polymères conducteurs d'électricité contenant des unités récurrentes dérivées de ces thiophènes substitués, ainsi qu'un procédé pour la fabrication des thiophènes substitués et un procédé pour la fabrication des polymères et les dispositifs électroconducteurs contenant ces polymères.

Dans la demande de brevet européen EP A 203438 (Allied Corporation), on a décrit des polymères conducteurs d'électricité dérivés de monomères de formule générale :

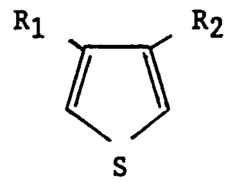

dans laquelle $R_1$ peut représenter entre autres un groupement alkyle substitué par un groupement époxy, halogéné, acide carboxylique et $R_2$ représente un atome d'hydrogène ou un radical méthyle. La demande française FR A 2 642 126, également déposée par Solvay SA, décrit des polymères conducteurs qui dérivent de monomères héterocycliques.

Il est notamment fait mention du thiophène substitué en position 3 par une chaîne du type $(CH_2)_m\{O-(CH_2)_2\}_nO-(CH_2)_p-CH_3$.

Toutefois, certaines applications électriques - telles que la réalisation de dispositifs basés sur l'èlectrochromisme (écrans d'affichage, commutateurs, éléments de mémoire...) impliquant une modification des propriétés d'absorption ou de transmission de la lumière du matériau mis en oeuvre, induite par une variation de la tension externe appliquée; la réalisation d'électrodes de batteries rechargeables, de cellules photovoltaïques, de cellules électrochimiques; la réalisation des dispositifs d'absorption des ondes électromagnétiques, etc. - exigent des polymères conducteurs aux propriétés particulières.

Ces propriétés particulières sont notamment la réversibilité électrochimique la plus complète, la stabilité la plus élevée possible du cycle d'oxydo-réduction entre les formes oxydée et réduite du système polymère-agent dopant, une variation importante des caractéristiques spectrales obtenue avec une variation de potentiel la plus faible possible, une bonne conductivité électrique et des absorptions importantes dans le domaine des rayonnements infrarouges proches et des hautes fréquences.

La présente invention vise à fournir une nouvelle famille de thiophènes substitués permettant notamment d'obtenir des polymères conducteurs d'électricité qui présentent les propriétés particulières susmentionnées à un degré élevé.

L'invention concerne à cet effet des monomères dérivés de thiophènes substitués de formule générale :

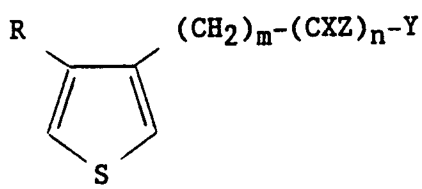

(I)

dans laquelle :

- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique au moins partiellement fluoré,
- m représente un nombre entier tel que $1 \leqq m \leqq 5$,
- n représente un nombre entier tel que $0 \leqq n \leqq 12$.

Habituellement :

- R représente un atome d'hydrogène,
- X et Z représentent un atome d'hydrogène ou un atome de fluor,

- Y représente :
  . un radical phényle substitué au moins par un atome de fluor, par un groupement $-CF_3$, un groupement $-CH_2F$ ou un groupement $CHF_2$, ou
  . un groupement aliphatique de formule générale $(CF_2)_p-CF_3$ dans laquelle p représente un nombre entier tel que $0 \leqq p \leqq 16$,
- m représente un nombre entier égal ou supérieur à 1,
- n représente un nombre entier tel que $0 \leqq n \leqq 12$.

Généralement :
- R représente un atome d'hydrogène,
- X et Z représentent un atome de fluor ou un atome d'hydrogène,
- Y représente :
  . un radical phényle substitué par un atome de fluor, par un groupement $-CF_3$ ou entièrement substitué par des atomes de fluor, ou
  . un groupement aliphatique de formule générale $-(CF_2)_p-CF_3$ dans laquelle p représente un nombre entier tel que $0 \leqq p \leqq 12$,
- n représente un nombre entier tel que $0 \leqq n \leqq 10$.

De manière préférée :
- R représente un atome d'hydrogène,
- X et Z représentent un atome de fluor ou un atome d'hydrogène,
- Y représente un groupement aliphatique de formule générale
- $(CF_2)_p-CF_3$ dans laquelle p représente un nombre entier tel que $1 \leqq p \leqq 8$,
- m est égal à 2 ou 3,
- n est égal à 0 ou 1.

De manière particulièrement préférée :
- R représente un atome d'hydrogène,
- X et Z représentent un atome de fluor,
- Y représente un groupement aliphatique de formule générale
- $(CF_2)_p-CF_3$ dans laquelle p représente un nombre entier égal à 2, 3 ou 4,
- m est égal à 3,
- n est égal à 1.

Les thiophènes substitués selon l'invention peuvent être synthétisés selon diverses méthodes.

Plusieurs méthodes peuvent être utilisées, telles qu'exemplifiées selon les schémas (1) et (2) de réaction des composés de formules générales suivantes, dans lesquelles R, X, Z, Y, m et n sont tels que définis précédemment et m' = (m-1), :

(1)

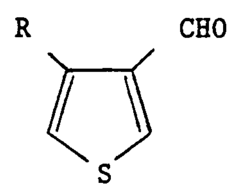

soit, avec $I-(CH_2)_{m'}-(CXZ)_n-Y$
ou $Br-(CH_2)_{m'}-(CXZ)_n-Y$
en présence de zinc, de magnésium, de manganèse, de cadmium et de préférence en présence de zinc, set en présence de pyridine, à 20 °C
soit en présence de $Y-(CXZ)_{n-}(CH_2)_{m'}-Mg-I$ et d'éther éthylique

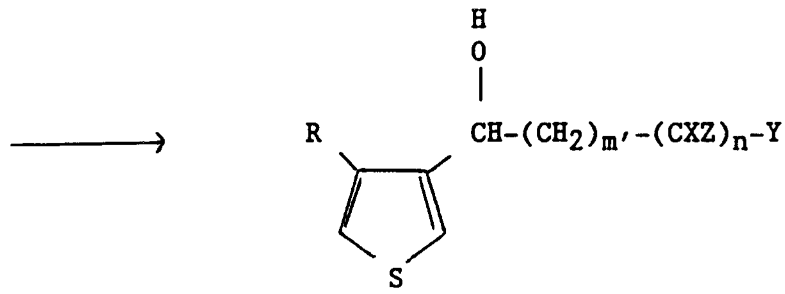

en présence de $SOCl_2$, $CHCl_3$ à reflux

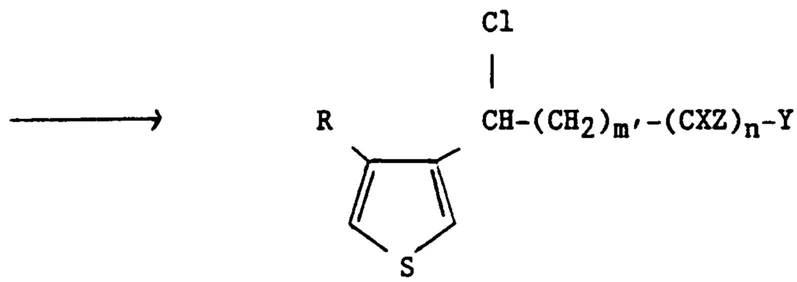

en présence de $LiAlH_4$, $PdCl_2$ et de tétrahydrofurane

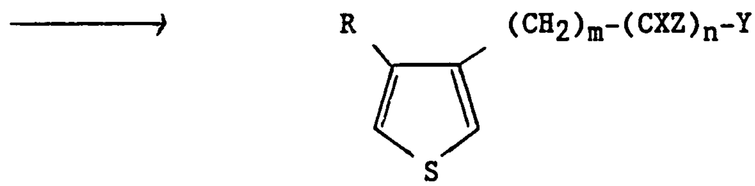

(2)

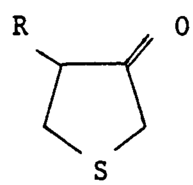

en présence de $Y(CXZ)_n-(CH_2)_mMg-I$ et d'éther éthylique

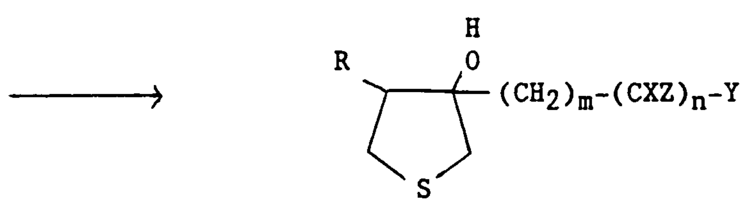

en présence de chlorure de tosyle et de pyridine

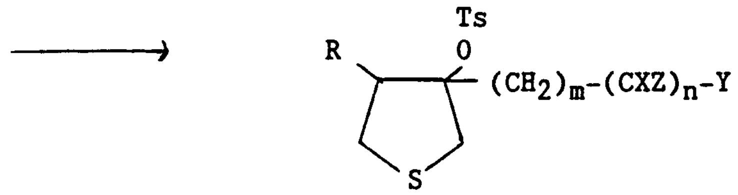

en présence de $KHSO_4$ et de soufre à 180 ° C

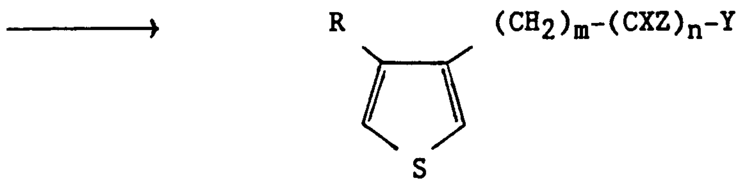

La température à laquelle sont réalisées ces réactions, est généralement comprise entre 0 et 200 ° C.

La pression à laquelle sont réalisées ces réactions, est généralement comprise entre 1 et 4 bars et de préférence ces réactions sont réalisées à la pression atmosphérique.

Les réactions sont réalisées de préférence sous l'atmosphère d'un gaz inerte, tel que notamment l'argon, et en présence d'un solvant, tel que notamment le tétrahydrofurane, et peuvent être réalisées dans tout réacteur ou appareillage permettant de réunir les conditions susmentionnées.

Un autre objet de l'invention est constitué par les polymères contenant des unités récurrentes dérivées de thiophène substitué selon l'invention.

L'invention concerne à cet effet des polymères de formule générale :

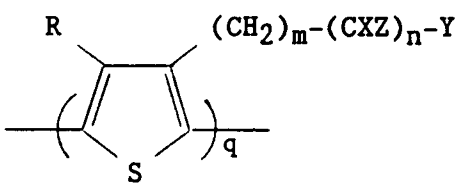

(II)

dans laquelle :

- q représente un nombre entier,
- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique au moins partiellement fluoré,
- m représente un nombre entier tel que $1 \leqq m \leqq 5$,
- n représente un nombre entier tel que $0 \leqq n \leqq 12$.

Habituellement :

- q représente un nombre entier compris entre 2 et 5000,
- R représente un atome d'hydrogène,
- X et Z représentent un atome d'hydrogène ou un atome de fluor,
- Y représente :
  . un radical phényle substitué au moins par un atome de fluor, par un groupement $-CF_3$, un groupement $-CH_2F$ ou un groupement $CHF_2$, ou
  . un groupement aliphatique de formule générale $(CF_2)_p-CF_3$ dans laquelle p représente un nombre entier tel que $0 \leqq p \leqq 16$,
- m représente un nombre entier égal ou supérieur à 1,
- n représente un nombre entier tel que $0 \leqq n \leqq 12$.

Généralement :

- q représente un nombre entier compris entre 2 et 3000,
- R représente un atome d'hydrogène,
- X et Z représentent un atome de fluor ou un atome d'hydrogène,
- Y représente :
  . un radical phényle substitué par un atome de fluor, par un groupement $-CF_3$ ou entièrement substitué par des atomes de fluor, ou
  . un groupement aliphatique de formule générale $(CF_2)_p-CF_3$ dans laquelle p représente un nombre entier tel que $0 \leqq p \leqq 12$,
- n représente un nombre entier tel que $0 \leqq n \leqq 10$.

De manière préférée :
- q représente un nombre entier compris entre 2 et 1000,
- R représente un atome d'hydrogène,
- X et Z représentent un atome de fluor ou un atome d'hydrogène
- Y représente un groupement aliphatique de formule générale $(CF_2)_p-CF_3$ dans laquelle p représente un nombre entier tel que $1 \leqq p \leqq 8$,
- m est égal à 2 ou 3,
- n représente un nombre entier égal à 0 ou 1.

De manière particulièrement préférée :
- q représente un nombre entier compris entre 2 et 500,
- R représente un atome d'hydrogène,
- X et Z représentent un atome de fluor,
- Y représente un groupement aliphatique de formule générale $(CF2)_p-CF_3$ dans laquelle p représente un nombre entier égal à 2, 3 ou 4,
- m est égal à 3,
- n est égal à 1.

De bons résultats ont été obtenus avec le poly (1H,1H,2H,2H,3H,3H perfluoroheptyl-3 thiophène) et le poly (1H,1H,2H,2H,3H,3H-perfluorononyl-3 thiophène).

Un autre objet de l'invention est constitué par les polymères conducteurs d'électricité contenant un polymère selon l'invention et un agent dopant. L'agent dopant peut être un anion ou un cation, tel que défini ci-après.

La préparation des polymères conducteurs d'électricité selon l'invention peut s'effectuer par voie chimique, en présence d'oxydants par exemple, ou par voie électrochimique. De bons résultats ont été obtenus lorsqu'on procède par polymérisation électrochimique, généralement dans une cellule d'électrolyse, par oxydation anodique du monomère au sein d'un solvant polaire et en présence d'électrolytes appropriés suivant des techniques conventionnelles telles que décrites notamment dans la demande de brevet français FA-A-2527843.

Selon ces techniques, la concentration en monomères est généralement comprise entre $10^{-3}$ et 1 mole par litre de solvant.

La température, à laquelle est réalisée la préparation des polymères, est généralement comprise entre 0 et 50 °C et, de préférence, entre 5 et 40 °C.

La pression, à laquelle est réalisée la préparation des polymères, est généralement voisine de la pression atmosphérique et, de préférence, est égale à la pression atmosphérique.

Comme solvants, on utilise de préférence des solvants polaires possédant des propriétés dissolvantes à la fois vis-à-vis du monomère et de l'électrolyte choisi et stables dans le domaine des potentiels appliqués. Des exemples de solvants utilisables sont l'acétonitrile, le chlorure de méthylène, le nitrobenzène et le carbonate de propylène.

Les électrolytes sont généralement choisis parmi les sels conducteurs de formule $C^+A^-$ dans laquelle $C^+$ est un cation et dans laquelle $A^-$ est un anion.

Le cation $C^+$ est choisi de préférence parmi les ions alcalins, les ions $R_4N^+$ et $R_4P^+$ (R étant un radical alkyle, tel que les radicaux éthyle et butyle par exemple).

L'anion $A^-$ est choisi de préférence parmi les ions $ClO_4^-$, $AsF_6^-$, $SbF_6^-$, $C_6H_5SO_3^-$, $BF_4^-$, $PF_6^-$ et $CF_3SO_3^-$.

Des électrolytes typiques sont par exemple les fluorophosphates, tels que l'hexafluorophosphate de tétrabutylammonium, les fluoroborates, tels que le tétrafluoroborate de tétraéthylammonium et les perchlorates, tels que le perchlorate de lithium et le perchlorate de tétrabutylammonium.

La concentration en électrolyte est généralement comprise entre $10^{-3}$ et 1 mole par litre de solvant.

La cellule électrochimique au sein de laquelle peut s'effectuer la polymérisation des monomères selon l'invention peut fonctionner dans des conditions potentiostatiques ou galvanostatiques.

Dans le premier cas (contrôle potentiostatique), la cellule comprend, outre la source de courant externe, trois électrodes dont une électrode de référence de contrôle du potentiel.

Au cours de l'électrolyse, une couche de polymère se dépose sur l'élément conducteur utilisé comme anode de la cellule d'électrolyse. Cette anode peut être réalisée en un métal noble, tel que l'or ou le platine, ou en un autre métal, tel que le cuivre, doré ou platiné, le titane, le nickel ou en un verre conducteur (oxyde d'étain, oxydes d'indium - étain). Après l'électrolyse, on dispose donc en fait d'une électrode constituée par un corps conducteur enrobé par un film de polymère y adhérant et qui contient une certaine proportion de l'anion provenant de l'électrolyte. Le polymère et l'anion forment ainsi un complexe à transfert de charges. La composition chimique du film de polymère peut être représentée par la formule empirique $(M^{+}Ay^{-})_q$ où $M^{+}$ représente le monomère, $A^{-}$ l'anion ou contre-ion, y la proportion en anion dans le polymère exprimée par unité monomérique (c'est-à-dire le taux de dopage) qui, dans le cas des polymères de l'invention, peut atteindre la valeur de 0,5, et q le degré de polymérisation généralement difficile à déterminer.

La polymérisation électrochimique du monomère s'effectuant sur l'anode de la cellule d'électrolyse, on ne peut pas obtenir directement une électrode recouverte d'un polymère dopé par des cations.

On peut, pour obtenir une telle électrode (cathode), se servir de l'anode obtenue précédemment et lui faire subir une double réduction. Une première réduction électrochimique est possible juste après la polymérisation en laissant l'anode dans la cellule d'électrolyse et en provoquant la décharge de la cellule. Cette décharge provoque l'extraction des anions "dopant" le polymère. On peut ensuite effectuer une seconde réduction sous atmosphère inerte, soit par voie chimique, soit par vole électrochimique. La voie chimique consiste à immerger le polymère dans une solution contenant les cations désirés. Ainsi, pour obtenir un polymère "dopé" par exemple par les cations $Li^{+}$, $Na^{+}$ ou $K^{+}$, on peut se servir par exemple d'une solution de naphtalène lithium, de naphtalène sodium ou de naphtalène potassium dans le tétrahydrofurane. La voie électrochimique consiste généralement à placer l'électrode en tant que cathode dans une cellule d'électrolyse contenant en solution les cations désirés. Les cations peuvent être par exemple des ions alcalins tels que ceux mentionnés ci-dessus, de préférence les cations $Li^{+}$ ou $K^{+}$, ou des ions complexes tels que $(Bu)_4N^{+}$ ou $(Et)_4N^{+}$ issus d'un électrolyte (de préférence $LiClO_4$, $KPF_6$, $(Bu)_4NClO_4$ et $(Et)_4NClO_4$) en solution dans un solvant tel que l'acétonitrile ou le carbonate de propylène. La concentration en électrolyte dans la solution est généralement comprise entre $10^{-3}$ et 1 mole pour 1 litre de solvant.

Les polymères conducteurs selon l'invention présentent un ensemble de propriétés tout à fait remarquables qui sont principalement :

- une réversibilité et une stabilité excellentes du cycle d'oxydo-réduction entre leurs formes oxydées et réduites;
- une variation importante des caractéristiques spectrales obtenue avec une variation de potentiel faible, ce qui rend intéressante et économique leur utilisation en tant que matériau électrochromique;
- une bonne conductivité électrique, généralement comprise entre 1 et $2.10^{2}$ $S.cm^{-1}$;
- des absorptions importantes dans le domaine des rayonnements infrarouges proches et à haute fréquence.

Les polymères conducteurs selon l'invention ont une bonne stabilité thermique et chimique et ont des propriétés d'hydrophobicité et de biocompatibilité. Le poly (1H,1H,2H,2H,3H,3H-perfluoroheptyl-3 thiophène) possède une élasticité supérieure à 30%.

Ces propriétés remarquables des polymères conducteurs selon l'invention les rendent particulièrement utilisables pour la réalisation de dispositifs électroconducteurs dont le principe de fonctionnement est basé sur ces propriétés, et qui constituent également un objet de la présente invention.

A titre d'exemples de dispositifs électroconducteurs contenant des polymères conducteurs dérivés des monomères hétérocycliques aromatiques substitués selon l'invention, on peut citer :

- les dispositifs électrochimiques de stockage d'énergie, tels que batteries d'accumulateurs et piles rechargeables ou non, dont les anodes (respectivement les cathodes) sont constituées d'électrodes revêtues de films desdits polymères dopés par des anions (respectivement des cations);
- les dispositifs électrochromiques basés sur la modification du spectre optique desdits polymères selon leur état d'oxydation, qui se manifeste lors des cycles d'oxydation et de réduction des films de polymères déposés sur les anodes (respectivement les cathodes) de ces dispositifs lors de la charge et de la décharge; à titre d'exemples de dispositifs, on peut citer les écrans d'affichage (display), les dispositifs optoélectroniques, les mémoires et commutateurs optiques;
- les dispositifs d'absorption des ondes électromagnétiques.

L'invention est illustrée par les exemples suivants.

Exemple 1 -

Synthèse du 1H,1H,2H,2H,3H,3H-perfluorononyl-3-thiophène

a) Synthèse de l'alcool

Dans un ballon à trois cols de 100 $cm^3$ équipé d'un réfrigérant surmonté d'une garde au chlorure de calcium, d'une admission d'argon, d'une ampoule à brome et d'un thermomètre, on introduit une solution de 2,9 g (25,8 mmol) de (3-thienyl)-méthanal dans 20 $cm^3$ d'éther éthylique.

Puis, on introduit dans l'ampoule à brome une solution d'iodure de 1H,1H,2H,2H-perfluorooctylmagnésium que l'on a préalablement préparé en mélangeant 0,65 g (0,027 atome.$g^{-1}$) de magnésium, 11,9 g (0,025 atome.$g^{-1}$) d'iodure de 1H,1H,2H,2H-perfiuorooctyle et 20 $cm^3$ d'éther éthylique.

Puis, on refroidit le ballon à 5°C et on introduit goutte à goutte la solution de magnésien dans le ballon.

Le mélange réactionnel ainsi obtenu est agité à température ambiante pendant 12 heures et est ensuite hydrolysé par 20 ml d'une solution d'acide chlorhydrique 2N.

On extrait la phase organique par 10 ml d'une solution à 38 % de $NaHSO_3$ 4 fois, puis par 2 fois 20 ml d'eau. Puis on sèche la phase organique au sulfate de magnésium et on l'évapore sous vide. On obtient une huile jaune qui est purifiée sur silice avec l'heptane-acétate d'éthyle 7/3 (en volume) comme éluant.

On récupère 10,0 g d'une huile incolore qui cristallise.

On obtient finalement 5,94 g d'alcool pur [thiényl (1H,1H,2H,2H-perfluorooctyl)méthanol] avec un rendement de 52 %.

b) Synthèse du composé chloré

On mélange 4,5 g (0,01 mol) de cet alcool (p.m. = 459,25), 5 $cm^3$ de chloroforme et 2 ml (27 mmol) de chlorure de thionyle.

On maintient ce mélange réactionnel à reflux pendant une heure.

Puis, on l'évapore sous vide, on le reprend par 2 fois 10 ml de méthanol et on l'évapore à nouveau sous vide. Puis on le reprend par 20 ml de chloroforme.

Ensuite, on le lave jusqu'à neutralité par une solution saturée de bicarbonate de sodium.

On obtient une huile incolore avec un rendement de 95 % [chloro-1H,1H,2H,2H,3H,3H-perfluorononyl-3 thiophène] (p.m. = 478,7).

c) Synthèse du thiophène fluoré

Dans un ballon à trois cols de 100 $cm^3$ équipé d'un réfrigérant surmonté d'une garde au chlorure de calcium, d'une admission d'argon et d'un dispositif d'introduction de poudres (chaussette), on introduit 4,5 g (9,5 mmol) du composé chloré obtenu, 2 g (11,3 mmol) de $PdCl_2$ anhydre et 10 ml de tétrahydrofurane anhydre.

Puis, on introduit lentement 0,4 g (10,5 mmol) d'hydrure de lithium et d'aluminium après avoir purgé à l'argon.

On agite le mélange réactionnel durant 2 heures à température ambiante. Puis, on le verse lentement sur de la glace pilée et on le laisse au repos durant 12 heures à 20°C.

Ensuite, le palladium est décanté et la phase organique est extraite par l'éther éthylique. On la sèche sur sulfate de magnésium.

Ensuite on évapore sous vide.

On obtient une huile colorée.

On la purifie sur une colonne de silice avec du pentane comme éluant.

On évapore le solvant.

On obtient 3,7 g d'une huile incolore qui cristallise à -4°C, le rendement est de 80 %.

On obtient le 1H,1H,2H,2H,3H,3H-perfluorononyl-3 thiophène avec un rendement global de 50 %.

Exemple 2

La synthèse du polymère est effectuée dans une cellule électrochimique thermostatée de 50 $cm^3$ à un compartiment contenant :

- $1.10^{-1}$ mole de monomère préparé comme à l'exemple 1,
- $2.10^{-2}$ mole d'hexafluorophosphate de tétrabutylammonium (fourni par Fluka),

- 25 ml de nitrobenzène distillé.

Les dépôts de polymère sont réalisés à 20 ° C, sous atmosphère d'argon, après dégazage de la solution par barbotage d'argon. Pour les caractéristiques électrochimiques, le polymère est déposé sur une électrode de platine massif d'une surface de 0,07 $cm^2$ polie. La quantité de charges utilisées est de 100 $mC/cm^2$ et la densité de courant est égale à 2 $mA/cm^2$. La cathode est constituée d'un fil de platine et l'électrode de référence est une électrode au calomel saturée.

La conductivité électrique du polymère ainsi obtenu est de l'ordre de 12 $S.cm^{-1}$.

Les propriétés électrochimiques des polymères ont été mesurées à partir du voltammogramme cyclique enregistré au moyen d'un potentiostat PAR modèle 173 et à partir des pics d'intensité enregistrés. La figure représente un voltammogramme réalisé avec un potentiel imposé de 1,8 V avec une vitesse de balayage de 20 mV/s dans 0,1 mole de perchlorate de lithium mis en solution dans $CH_3CN$, l'unité de l'abscisse est le voit (V), l'unité de l'ordonnée est le microampère ($\mu$A), le minimum ($Ep_c$) est à 0,76 V et le maximum ($Ep_a$) à 0,92 V..

Les rapports $Ip_a/Ip_c$ entre les intensités de courant d'oxydation ($Ip_a$) et de réduction ($Ip_c$) (pour le système p) sont d'environ 1,2.

Le dopage "p" du polymère est très réversible. La charge échangée après 5000 cycles entre -0,2 et 1,25 volt à 200 mV/s, est encore de 90 %, le polymère étant chaque fois dopé et dédopé à 10 %, soit 50% de la charge maximale échangée sous cyclage lent (20 m V/s).

## Revendications

**Revendications pour les Etats contractants suivants : AT, BE, CH, DE, DK, FR, GB, IT, LI, LU, NL, SE**

**1.** Thiophènes substitués de formule générale :

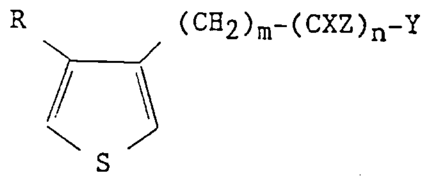

(I)

caractérisés en ce que :

- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique, au moins partiellement fluoré,
- m représente un nombre entier tel que $1 \leqq m \leqq 5$,
- n représente un nombre entier tel que $0 \leqq n \leqq 12$.

**2.** Thiophènes substitués selon la revendication 1, caractérisés en ce que :

- R représente un atome d'hydrogène,
- X et Z représentent un atome de fluor,
- Y représente un groupement aliphatique de formule générale $(CF_2)_p$-$CF_3$ dans laquelle p représente un nombre entier égal à 2, 3 ou 4,
- m est égal à 3,
- n est égal à 1.

**3.** Polymères de formule générale :

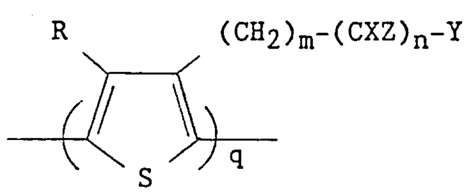

(II)

dans laquelle :

- q représente un nombre entier,
- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique, au moins partiellement fluoré,
- m représente un nombre entier tel que $1 \leqq m \leqq 5$,
- n représente un nombre entier tel que $0 \leqq n \leqq 12$.

**4.** Polymères selon la revendication 3, caractérisés en ce que :
- q représente un nombre entier compris entre 2 et 500,
- R représente un atome d'hydrogène,
- X et Z représentent un atome de fluor,
- Y représente un groupement aliphatique de formule générale $-(CF_2)_p-CF_3$ dans laquelle p représente un nombre entier égal à 2, 3 ou 4,
- m est égal à 3,
- n est égal à 1.

**5.** Polymères conducteurs d'électricité contenant un polymère selon la revendication 3 ou 4 et un agent dopant.

**6.** Procédé pour la fabrication de polymères conducteurs d'électricité selon la revendication 5, caractérisé en ce que l'on polymérise électrochimiquement, par oxydation anodique au sein d'un solvant polaire et en présence d'un électrolyte approprié, un monomère choisi parmi les thiophènes substitués selon la revendication 1 ou 2.

**7.** Dispositifs électroconducteurs contenant un polymère conducteur d'électricité selon la revendication 5.

**8.** Dispositifs électrochimiques de stockage d'énergie dont les anodes (respectivement les cathodes) sont constituées d'électrodes revêtues de films de polymères selon la revendication 3 ou 4 dopés par des anions (respectivement des cations).

**9.** Dispositifs électrochromiques dont les anodes (respectivement les cathodes) sont revêtues de films de polymères selon la revendication 5.

**10.** Dispositifs d'absorption des rayonnements infrarouges proches et à haute fréquence à base de polymères selon la revendication 5.

**Revendications pour les Etats contractants suivants : ES, GR**

**1.** Procédé pour la fabrication de polymères conducteurs d'électricité contenant un agent dopant et un polymère de formule générale :

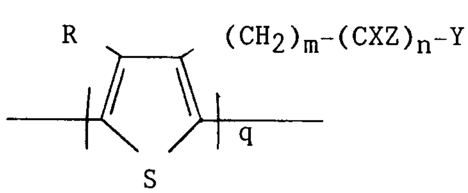

(II)

dans laquelle :
- q représente un nombre entier,
- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique, au moins partiellement fluoré,

- m représente un nombre entier tel que $1 \leq m \leq 5$,
- n représente un nombre entier tel que $0 \leq n \leq 12$,

caractérisé en ce que l'on polymérise électrochimiquement, par oxydation anodique au sein d'un solvant polaire et en présence d'un électrolyte approprié, un monomère choisi parmi les thiophènes substitués de formule générale :

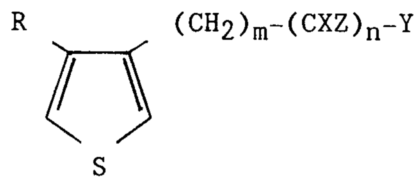

(I)

dans laquelle R, X, Z, Y, m et n sont tels que définis.

**2.** Procédé selon la revendication 1, caractérisé en ce que l'on polymérise un monomère choisi parmi les thiophènes substitués de formule générale (I) dans laquelle :
- R représente un atome d'hydrogène,
- X et Z représentent un atome de fluor,
- Y représente un groupement aliphatique de formule générale $-(CF_2)_p-CF_3$ dans laquelle p représente un nombre entier égal à 2, 3 ou 4,
- m est égal à 3,
- n est égal à 1.

**3.** Procédé pour la fabrication de thiophènes substitués de formule générale :

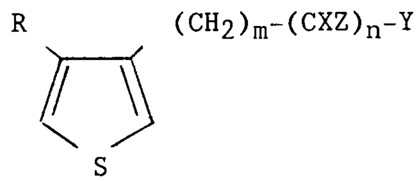

(I)

dans laquelle :
- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique, au moins partiellement fluoré,
- m représente un nombre entier tel que $1 \leq m \leq 5$,
- n représente un nombre entier tel que $0 \leq n \leq 12$, caractérisé en ce qu'il comprend les étapes suivantes :
- dans une première étape, on fait réagir le composé de formule générale (A) dans laquelle R, X, Z, Y, m, n sont tels que définis et m' : (m-1),

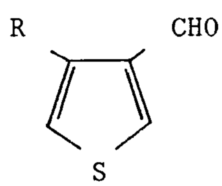

(A)

soit, avec $I-(CH_2)_{m'}-(CXZ)_n-Y$

ou $Br-(CH_2)_{m'}-(CXZ)_n-Y$

en présence de zinc, de magnésium, de manganèse ou de cadmium et en présence de pyridine, à 20 °C

soit en présence de $Y\text{-}(CXZ)_n\text{-}(CH_2)_{m'}\text{-}Mg\text{-}I$ et d'éther éthylique, en vue d'obtenir le composé de formule générale :

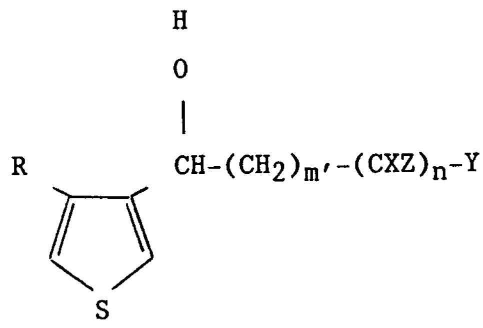

(B)

- dans une deuxième étape, on fait réagir le composé de formule générale (B) en présence de $SOCl_2$ et de $CHCl_3$ à reflux, en vue d'obtenir le composé de formule générale

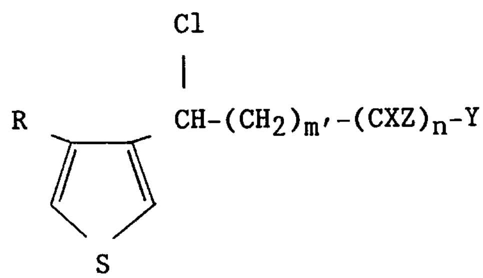

(C)

- dans une troisième étape, on fait réagir le composé de formule générale (C) en présence de $LiAlH_4$, $PdCl_2$ et de tétrahydrofurane

**4.** Procédé pour la fabrication de thiophènes substitués de formule générale :

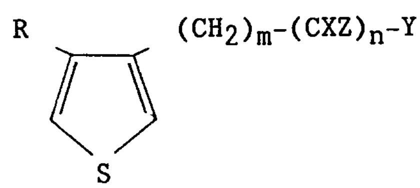

(I)

dans laquelle :

- R représente un atome d'hydrogène ou un groupement aliphatique contenant de 1 à 4 atomes de carbone,
- X et Z peuvent être identiques ou différents et représentent un atome d'hydrogène ou un atome de fluor,
- Y représente un groupement aliphatique ou aromatique, au moins partiellement fluoré,
- m représente un nombre entier tel que $1 \leqq m \leqq 5$,
- n représente un nombre entier tel que $0 \leqq n \leqq 12$, caractérisé en ce qu'il comprend les étapes suivantes :
- dans une première étape, on fait réagir le composé de formule générale :

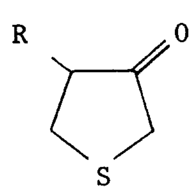

(D)

en présence de $Y\text{-}(CXZ)_n\text{-}(CH_2)_m\text{-}Mg\text{-}I$ et d'éther éthylique en vue d'obtenir le composé de formule générale :

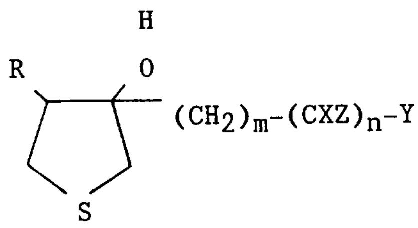

(E)

- dans une deuxième étape, on fait réagir le composé de formule générale (E) en présence de chlorure de tosyle et de pyridine en vue d'obtenir le composé de formule générale :

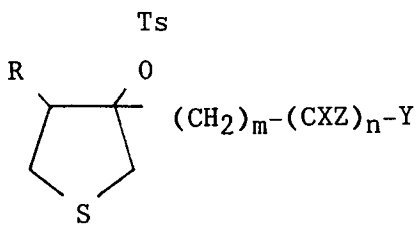

(F)

- dans une troisième étape, on fait réagir le composé de formule générale (F) en présence de $KHSO_4$ et de soufre à 180 ° C.

**5.** Dispositifs électroconducteurs contenant un polymère conducteur d'électricité obtenu selon la revendication 1 ou 2.

**6.** Dispositifs électrochimiques de stockage d'énergie dont les anodes (respectivement les cathodes) sont constituées d'électrodes revêtues de films de polymères obtenus selon la revendication 1 ou 2 dopés par des anions (respectivement des cations).

**7.** Dispositifs électrochromiques dont les anodes (respectivement les cathodes) sont revêtues de films de polymères obtenus selon la revendication 1 ou 2.

**8.** Dispositifs d'absorption des rayonnements infrarouges proches et à haute fréquence à base de polymères obtenus selon la revendication 1 ou 2.

**Claims**
**Claims for the following Contracting States : AT, BE, CH, DE, DK, FR, GB, IT, LI, LU, NL, SE**

**1.** Substituted thiophenes of general formula:

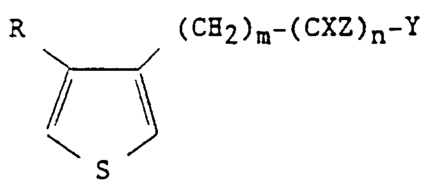

(I)

characterized in that:
- R represents a hydrogen atom or an aliphatic group containing from 1 to 4 carbon atoms,
- X and Z may be identical or different and represent a hydrogen atom or a fluorine atom,
- Y represents an at least partially fluorinated aliphatic or aromatic group,
- m represents an integer such that $1 \leqq m \leqq 5$,
- n represents an integer such that $0 \leqq n \leqq 12$,

**2.** Substituted thiophenes according to Claim 1, characterized in that:
- R represents a hydrogen atom,
- X and Z represent a fluorine atom,
- Y represents an aliphatic group of general formula $-(CF_2)_p-CF_3$ in which p represents an integer equal to 2, 3 or 4,
- m is 3, and
- n is 1.

**3.** Polymers of general formula:

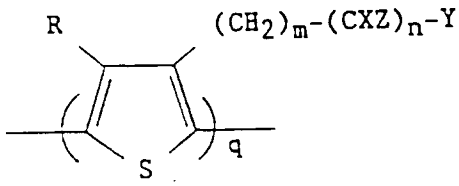

(II)

in which:
- q represents an integer,
- R represents a hydrogen atom or an aliphatic group containing from 1 to 4 carbon atoms,
- X and Z may be identical or different and represent a hydrogen atom or a fluorine atom,
- Y represents an at least partially fluorinated aliphatic or aromatic group,
- m represents an integer such that $1 \leqq m \leqq 5$,
- n represents an integer such that $0 \leqq n \leqq 12$,

**4.** Polymers according to Claim 3, characterized in that:
- q represents an integer between 2 and 500,
- R represents a hydrogen atom,
- X and Z represent a fluorine atom,
- Y represents an aliphatic group of general formula $-(CF_2)_p-CF_3$ in which p represents an integer equal to 2, 3 or 4,
- m is 3, and
- n is 1.

**5.** Electrically conducting devices containing a polymer according to Claim 3 or 4 and a doping agent.

**6.** Process for the production of electrically conducting polymers according to Claim 5, characterized in that a monomer chosen from the substituted thiophenes according to Claim 1 or 2 is polymerized electrochemically, by anodic oxidation in a polar solvent and in the presence of an appropriate electrolyte.

**7.** Electrically conducting devices containing an electrically conducting polymer according to Claim 5.

**8.** Electrochemical devices for storing energy, the anodes (or, respectively, the cathodes) of which consist of electrodes coated with films of polymers according to Claim 3 or 4 doped by anions (or, respectively, cations).

**9.** Electrochromic devices in which the anodes (or, respectively, the cathodes) are coated with films or polymers according to Claim 5.

**10.** Devices for the absorption of near infrared and high frequency radiation based on polymers according to Claim 5.

**Claims for the following Contracting States : ES, GR**

**1.** Process for the production of electrically conducting polymers containing a doping agent and a polymer of general formula:

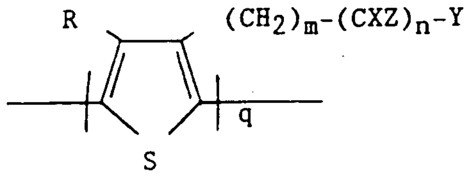

(II)

in which:
- q represents an integer,
- R represents a hydrogen atom or an aliphatic group containing from 1 to 4 carbon atoms,
- X and Z may be identical or different and represent a hydrogen atom or a fluorine atom,
- Y represents an at least partially fluorinated aliphatic or aromatic group,
- m represents an integer such that 1 ≦ m ≦ 5,
- n represents an integer such that 0 ≦ n ≦ 12, characterized in that a monomer chosen from the substituted thiophenes of general formula:

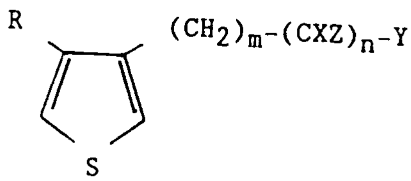

(I)

in which R, X, Z, Y, m and n are as defined,
is polymerized electrochemically, by anodic oxidation in a polar solvent and in the presence of an appropriate electrolyte.

**2.** Process according to Claim 1, characterized in that a monomer chosen from the substituted thiophenes of general formula (I) in which:
- R represents a hydrogen atom,
- X and Z represent a fluorine atom,
- Y represents an aliphatic group of general formula $-(CF_2)_p-CF_3$ in which p represents an integer equal to 2, 3 or 4,
- m is 3, and
- n is 1

is polymerized.

**3.** Process for the production of substituted thiophenes of general formula:

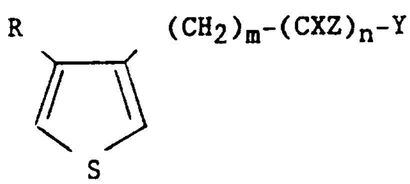

(I)

in which:
- R represents a hydrogen atom or an aliphatic group containing from 1 to 4 carbon atoms,
- X and Z may be identical or different and represent a hydrogen atom or a fluorine atom,
- Y represents an at least partially fluorinated aliphatic or aromatic group,
- m represents an integer such that 1 ≦ m ≦ 5,
- n represents an integer such that 0 ≦ n ≦ 12, characterized in that it comprises the following stages:
- in a first stage, the compound of general formula (A) in which R, X, Z, Y, m and n are as defined and m': (m-1),

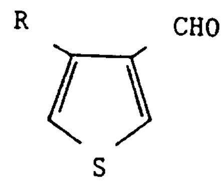

(A)

is reacted
either with I-$(CH_2)_{m'}$-$(CXZ)_n$-Y
or Br-$(CH_2)_{m'}$-$(CXZ)_n$-Y
in the presence of zinc, magnesium, manganese or cadmium and in the presence of pyridine, at 20 °C,
or in the presence of Y-$(CXZ)_n$-$(CH_2)_{m'}$-Mg-I and ethyl ether, with a view to obtaining the compound of general formula:

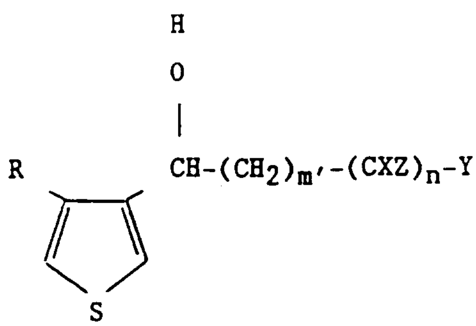

(B)

- in a second stage, the compound of general formula (B) is reacted in the presence of $SOCl_2$ and of $CHCl_3$ at reflux, with a view to obtaining the compound of general formula

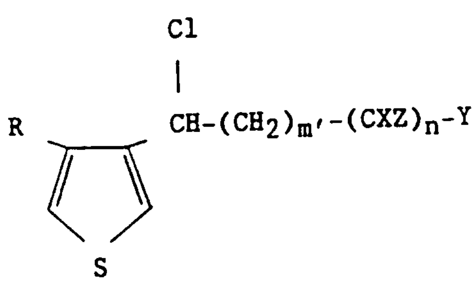

(C)

- in a third stage, the compound of general formula (C) is reacted in the presence of $LiAlH_4$, $PdCl_2$ and tetrahydrofuran.

**4.** Process for the production of substituted thiophenes of general formula:

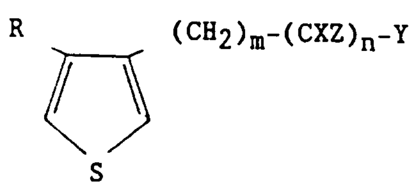

(I)

in which:

- R represents a hydrogen atom or an aliphatic group containing from 1 to 4 carbon atoms,
- X and Z may be identical or different and represent a hydrogen atom or a fluorine atom,

- Y represents an at least partially fluorinated aliphatic or aromatic group,
- m represents an integer such that 1 ≦ m ≦ 5,
- n represents an integer such that 0 ≦ n ≦ 12, characterized in that it comprises the following stages:
- in a first stage, the compound of general formula:

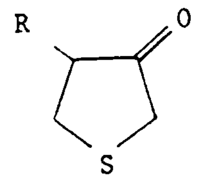

(D)

is reacted in the presence of Y-$(CHZ)_n$-$(CH_2)_m$-Mg-I and ethyl ether, with a view to obtaining the compound of general formula:

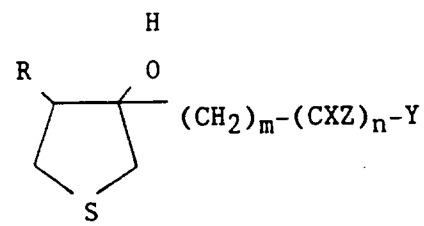

(E)

- in a second stage, the compound of general formula (E) is reacted in the presence of tosyl chloride and pyridine, with a view to obtaining the compound of general formula:

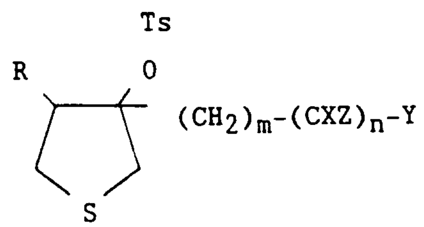

(F)

- in a third stage, the compound of general formula (F) is reacted in the presence of $KHSO_4$ and sulphur at 180 °C.

**5.** Electrically conducting devices containing an electrically conducting polymer obtained according to Claim 1 or 2.

**6.** Electrochemical devices for storing energy, the anodes (or, respectively, the cathodes) of which consist of electrodes coated with films of polymers obtained according to Claim 1 or 2 doped by anions (or, respectively, cations).

**7.** Electrochromic devices in which the anodes (or, respectively, the cathodes) are coated with films of polymers obtained according to Claim 1 or 2.

**8.** Devices for the absorption of near infrared and high frequency radiation based on polymers obtained according to Claim 1 or 2.

**Patentansprüche**

**Patentansprüche für folgende Vertragsstaaten : AT, BE, CH, DE, DK, FR, GB, IT, LI, LU, NL, SE**

**1.** Substituierte Thiophene der allgemeinen Formel:

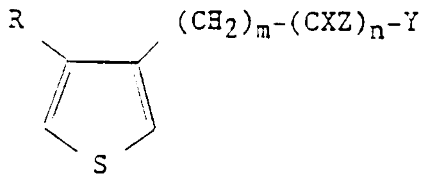

(I)

dadurch gekennzeichnet, daß:

- R ein Wasserstoffatom oder eine aliphatische Gruppe mit 1 bis 4 Kohlenstoffatomen darstellt,
- X und Z gleich oder verschieden sein können und ein Wasserstoffatom oder ein Fluoratom darstellen,
- Y eine wenigstens teilweise fluorierte aliphatische oder aromatische Gruppe darstellt,
- m eine ganze Zahl wie $1 \leqq m \leqq 5$ darstellt,
- n eine ganze Zahl wie $0 \leqq n \leqq 12$ darstellt.

**2.** Substituierte Thiophene gemäß Anspruch 1, dadurch gekennzeichnet, daß:

- R ein Wasserstoffatom darstellt,
- X und Z ein Fluoratom darstellen,
- Y eine aliphatische Gruppe der allgemeinen Formel $-(CF_2)_p-CF_3$ darstellt, worin p eine ganze Zahl gleich 2,3 oder 4 darstellt,
- m gleich 3 ist,
- n gleich 1 ist.

**3.** Polymere der allgemeinen Formel:

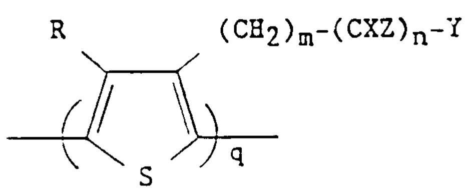

(II)

worin:

- q eine ganze Zahl darstellt,
- R ein Wasserstoffatom oder eine aliphatische Gruppe mit 1 bis 4 Kohlenstoffatomen darstellt,
- X und Z gleich oder verschieden sein können und ein Wasserstoffatom oder ein Fluoratom darstellen,
- Y eine wenigstens teilweise fluorierte aliphatische oder aromatische Gruppe darstellt,
- m eine ganze Zahl wie $1 \leqq m \leqq 5$ darstellt,
- n eine ganze Zahl wie $0 \leqq n \leqq 12$ darstellt.

**4.** Polymere gemäß Anspruch 3, dadurch gekennzeichnet, daß:

- q eine ganze Zahl zwischen 2 und 500 darstellt,
- R ein Wasserstoffatom darstellt,
- X und Z ein Fluoratom darstellen,
- Y eine aliphatische Gruppe der allgemeinen Formel $(CF_2)_p-CF_3$ darstellt, worin p eine ganze Zahl gleich 2,3 oder 4 darstellt,
- m gleich 3 ist,
- n gleich 1 ist.

**5.** Elektrisch leitfähiges Polymer, das ein Polymer gemäß Anspruch 3 oder 4 und ein Dotierungsmittel enthält.

6. Verfahren zur Herstellung elektrisch leitfähiger Polymere gemäß Anspruch 5, dadurch gekennzeichnet, daß man ein unter den substituierten Thiophenen gemäß Anspruch 1 oder 2 ausgewähltes Monomer durch anodische Oxidation in einem polaren Lösungsmittel und in Gegenwart eines geeigneten Elektrolyten elektrochemisch polymerisiert.

7. Elektrisch leitende Vorrichtungen, die ein elektrisch leitfähiges Polymer gemäß Anspruch 5 enthalten.

8. Elektrochemische Vorrichtungen zur Energiespeicherung, deren Anoden (beziehungsweise deren Kathoden) aus Elektroden gebildet werden, die mit Filmen der Polymere gemäß Anspruch 3 oder 4, die mit Anionen (beziehungsweise Kationen) dotiert sind, beschichtet sind.

9. Elektrochrome Vorrichtungen, deren Anoden (beziehungsweise Kathoden) mit Filmen der Polymere gemäß Anspruch 5 beschichtet sind.

10. Vorrichtungen zur Absorption von Strahlung des nahen Infrarot und von hoher Frequenz auf der Basis von Polymeren gemäß Anspruch 5.

**Patentansprüche für folgende Vertragsstaaten : ES, GR**

1. Verfahren zur Herstellung elektrisch leitfähiger Polymere, die ein Dotierungsmittel und ein Polymer der allgemeinen Formel:

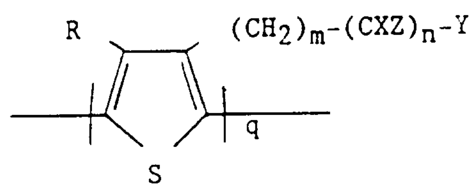

(II)

enthalten, worin:
- q eine ganze Zahl darstellt,
- R ein Wasserstoffatom oder eine aliphatische Gruppe mit 1 bis 4 Kohlenstoffatomen darstellt,
- X und Z gleich oder verschieden sein können und ein Wasserstoffatom oder ein Fluoratom darstellen,
- Y eine wenigstens teilweise fluorierte aliphatische oder aromatische Gruppe darstellt,
- m eine ganze Zahl wie $1 \leqq m \leqq 5$ darstellt,
- n eine ganze Zahl wie $0 \leqq n \leqq 12$ darstellt,

dadurch gekennzeichnet, daß man ein Monomer, das unter den substituierten Thiophenen der allgemeinen Formel:

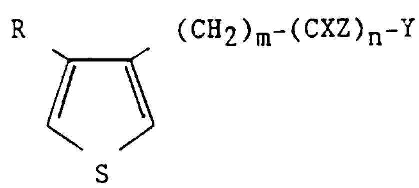

(I)

worin R, X, Z, Y, m und n so sind wie definiert, ausgewählt ist, durch anodische Oxidation in einem polaren Lösungsmittel und in Gegenwart eines geeigneten Elektrolyten elektrochemisch polymerisiert.

2. Verfahren gemäß Anspruch 1, dadurch gekennzeichnet, daß man ein Monomer polymerisiert, das unter den substituierten Thiophenen der allgemeinen Formel (I) ausgewählt ist, worin:
- R ein Wasserstoffatom darstellt,
- X und Z ein Fluoratom darstellen,
- Y eine aliphatische Gruppe der allgemeinen Formel $-(CF_2)_p-CF_3$ darstellt, worin p eine ganze Zahl gleich 2, 3 oder 4 darstellt,

- m gleich 3 ist,
- n gleich 1 ist.

**3.** Verfahren zur Herstellung substituierter Thiophene der allgemeinen Formel:

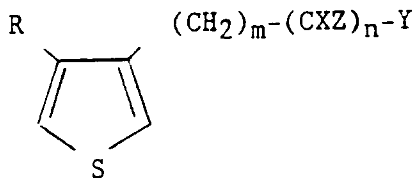

(I)

worin:
- R ein Wasserstoffatom oder eine aliphatische Gruppe mit 1 bis 4 Kohlenstoffatomen darstellt,
- X und Z gleich oder verschieden sein können und ein Wasserstoffatom oder ein Fluoratom darstellen,
- Y eine wenigstens teilweise fluorierte aliphatische oder aromatische Gruppe darstellt,
- m eine ganze Zahl wie $1 \leqq m \leqq 5$ darstellt,
- n eine ganze Zahl wie $0 \leqq n \leqq 12$ darstellt,

dadurch gekennzeichnet, daß es die folgenden Schritte umfaßt:
- in einem ersten Schritt setzt man die Verbindung der allgemeinen Formel (A), worin R, X, Z, Y, m, n so sind wie definiert und m' : (m-1),

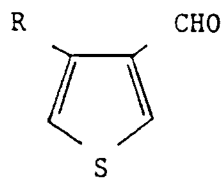

(A)

entweder mit $I-(CH_2)_{m'}-(CXZ)_n-Y$ oder $Br-(CH_2)_{m'}-(CXZ)_n-Y$ in Gegenwart von Zink, Magnesium, Mangan oder Cadmium und in Gegenwart von Pyridin bei 20 °C oder in Gegenwart von $Y-(CXZ)_n-(CH_2)_{m'}-Mg-I$ und Ethylether um, wobei die Verbindung der allgemeinen Formel:

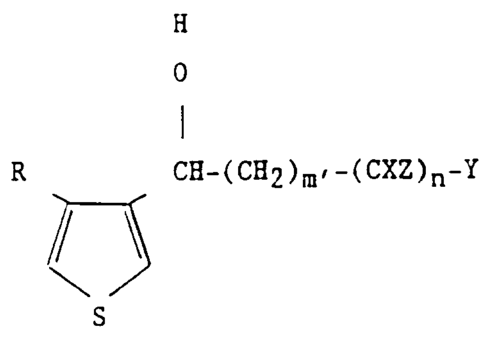

(B)

erhalten wird
- in einem zweiten Schritt setzt man die Verbindung der allgemeinen Formel (B) in Gegenwart von $SOCl_2$ und $CHCl_3$ unter Rückfluß um, wobei die Verbindung der allgemeinen Formel:

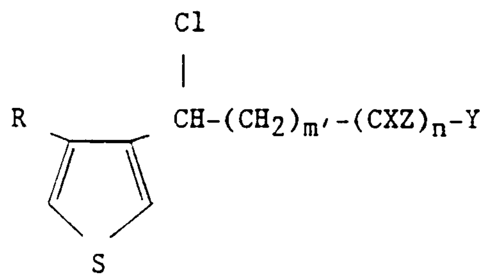

(C)

erhalten wird

- in einem dritten Schritt setzt man die Verbindung der allgemeinen Formel (C) in Gegenwart von $LiAlH_4$, $PdCl_2$ und Tetrahydrofuran um

**4.** Verfahren zur Herstellung substituierter Thiophene der allgemeinen Formel:

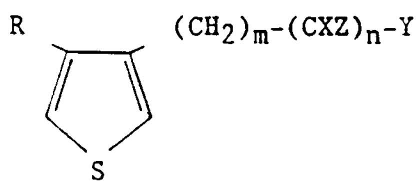

(I)

worin:

- R ein Wasserstoffatom oder eine aliphatische Gruppe mit 1 bis 4 Kohlenstoffatomen darstellt,
- X und Z gleich oder verschieden sein können und ein Wasserstoffatom oder ein Fluoratom darstellen,
- Y eine wenigstens teilweise fluorierte aliphatische oder aromatische Gruppe darstellt,
- m eine ganze Zahl wie $1 \leqq m \leqq 5$ darstellt,
- n eine ganze Zahl wie $0 \leqq n \leqq 12$ darstellt,

dadurch gekennzeichnet, daß es die folgenden Schritte umfaßt:

- in einem ersten Schritt setzt man die Verbindung der allgemeinen Formel:

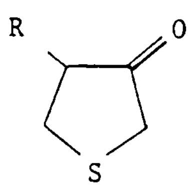

(D)

in Gegenwart Von $Y-(CXZ)_n-(CH_2)_m-Mg-I$ und Ethylether um, wobei die Verbindung der allgemeinen Formel:

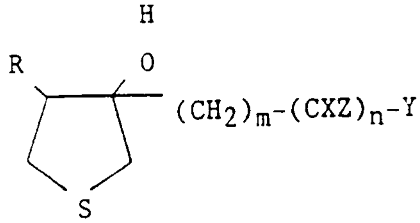

(E)

erhalten wird

- in einem zweiten Schritt setzt man die Verbindung der allgemeinen Formel (E) in Gegenwart von Tosylchlorid und Pyridin um, wobei die Verbindung der allgemeinen Formel:

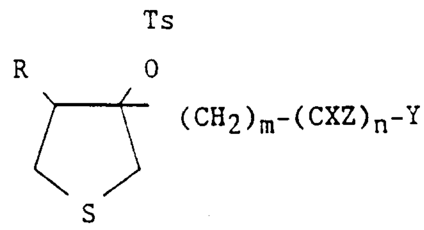

(F)

erhalten wird

- in einem dritten Schritt setzt man die Verbindung der allgemeinen Formel (F) in Gegenwart von $KHSO_4$ und Schwefel bei 180 °C um.

**5.** Elektrisch leitende Vorrichtungen, die ein gemäß Anspruch 1 oder 2 erhaltenes elektrisch leitfähiges Polymer enthalten.

**6.** Elektrochemische Vorrichtungen zur Energiespeicherung, deren Anoden (beziehungsweise deren Kathoden) aus Elektroden gebildet werden, die mit Filmen der gemäß Anspruch 1 oder 2 erhaltenen Polymere, die mit Anionen (beziehungsweise Kationen) dotiert sind, beschichtet sind.

**7.** Elektrochrome Vorrichtungen, deren Anoden (beziehungsweise Kathoden) mit Filmen der gemäß Anspruch 1 oder 2 erhaltenen Polymere beschichtet sind.

**8.** Vorrichtungen zur Absorption von Strahlung des nahen Infrarot und von hoher Frequenz auf der Basis von gemäß Anspruch 1 oder 2 erhaltenen Polymeren.

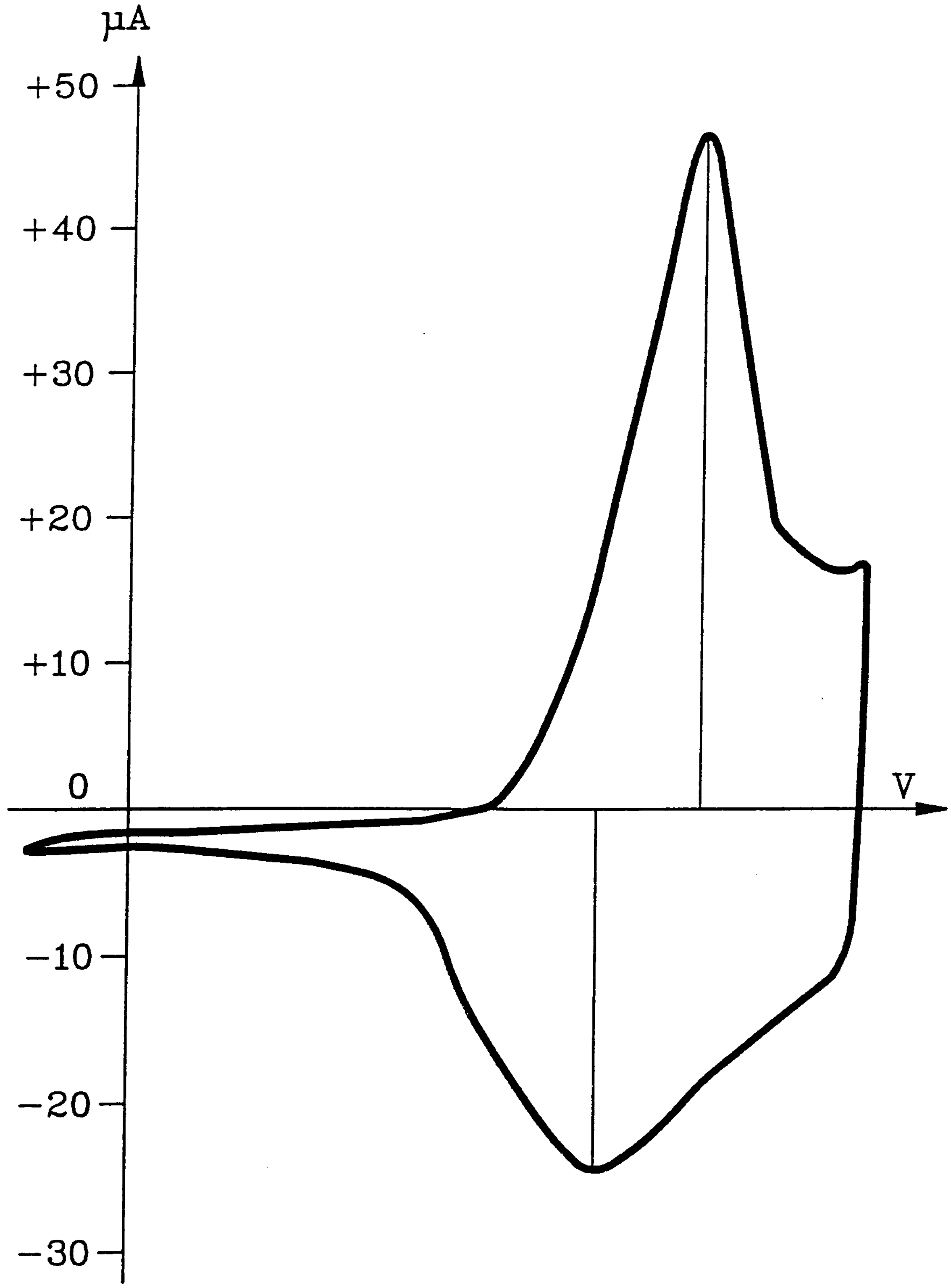
µA
+50
+40
+30
+20
+10
0
-10
-20
-30
V